Europäisches Patentamt

European Patent Office

Office européen des brevets

(11) Publication number: **0 288 633**
**A2**

# EUROPEAN PATENT APPLICATION

(21) Application number: **87304337.6**

(51) Int. Cl.⁴: **A61L 9/01**

(22) Date of filing: **15.05.87**

(30) Priority: **27.04.87 US 42668**

(43) Date of publication of application:
**02.11.88 Bulletin 88/44**

(84) Designated Contracting States:
**BE DE FR GB NL SE**

(71) Applicant: **AQUASCIENCE RESEARCH GROUP, INC.**
**1100 Gentry**
**North Kansas City Missouri 64116(US)**

(72) Inventor: **Kuhns, John Farrell**
**7601 E. Forest Lake Drive**
**Parkville, Missouri(US)**

(74) Representative: **Perry, Robert Edward et al**
**GILL JENNINGS & EVERY 53-64 Chancery Lane**
**London WC2A 1HN(GB)**

(54) **Odour control.**

(57) A process for controlling an odour of a nitrogenous or sulfur-containing compound, a musk and/or an odour of fecal, urinary or glandular origin, which comprises applying to the odour, its source or the area of its source a bisulfite selected from sodium formaldehydebisulfite and potassium formaldehydebisulfite, or material which reacts to form the bisulfite in situ. Preferably, the bisulfite is sodium formaldehydebisulfite applied directly to the odour-causing substance or to any substrate carrying the odour.

EP 0 288 633 A2

0 288 633

## ODOUR CONTROL

This invention relates to the control of odour. More specifically, the invention relates to removing odours caused by ammoniacal substances (ammonia, amines, amides) and other nitrogenous substances, sulfur-containing substances such as mercaptans and thiols, and various musks. The invention involves the use of products of the type disclosed in EP-A-0203741 entitled "Method and Product for Removal of Chloramines, Chlorine and Ammonia from Aquaculture Water".

Since the beginning of time, man has been confronted with the problem of offensive odours, and has attempted either to eliminate or to cover (mask) the odour. It has been difficult to eliminate many odours. Thus, the most common solution to offensive odour problems has been to cover, or mask the offensive odour with another odour that is considered acceptable and non-offensive. This solution is seen in the commercial world in such products as aerosol air fresheners and solid room deodorants that are scented with an odour that the public finds pleasing. Many other products are scented not only to create a pleasant odour or aroma, but to mask an existing odour that is not considered pleasant. This is obviously a very profitable business as evidenced by the numerous products that are available for this purpose. But these types of products do not "cure" the odor problem. At best they offer a temporary solution. Once the "masking" odor has dissipated, it must be reapplied to continue hiding the offensive odor. Also, some odors are exceptionally strong and pervasive requiring an enormous amount of the masking odor, which in such large quantities, can become almost as offensive as the original odor. The primary drawback of the "masking odor" solution to odor control is that it does not eliminate the original odor. A product that eliminates the original offensive odor would create a permanent and continuing remedy to the problem and not present the disadvantage present in the "masking" remedy of continual reapplication for continued effectiveness.

Possibly the most offensive odors, to man, are those odors associated with fresh, aged, or decomposing matter of fecal, urinary, and glandular origin. This includes such products from man and animals. Another offensive odor is that associated with the human body. Body odors are produced by bacteria metabolizing secretions from various skin glands. A third odor of universal concern is that associated with chemical factories that create by-products or waste-products containing ammonia or sulfur compounds.

A product that would effectively eliminate these odors would be desirable for numerous reasons. First, it would create a lasting remedy of the problem of offensive odors. Second, it would be economically advantageous to be able to eliminate an odor once and for all time as opposed to a continual attempt to mask the odor. Third, especially as to extremely offensive odors, immediate elimination would avoid the problem of using very strong "masking" odors or large quantities of such "masking" odors. Fourth, a product that initially eliminates the odor creates more economic efficiency in that there is no need to produce an array of "masking" odor products in varying scents to satisfy differing individual's idea of what is an acceptable odor. Fifth, elimination of offensive odors creates a more healthful environment for those subjected to the odor. Sixth, elimination of the odor, and a chemical breakdown of the odor causing substances, may reduce the possibilities of diseases caused by the constituent parts.

Odors created by products of fecal, urinary and glandular origin often contain substances containing ammonia, amines, amides and other nitrogenous substances, mercaptans, thiols, and other sulfur-containing substances and musks. Those products derived from the waste products (or by-products) of metabolic systems (either man or animal) contain many odor creating substances of the type mentioned (metabolized amino acids will produce ammonia, as well as ammonia and amine precursors) and numerous others.

An area where the odor problem is most acute and the desire for effective control is most needed is in products associated with animal litter and bedding. Whether the animal litter is being used on a large scale as in a barn, or on an intermediate scale as in a pet shop, or on a small scale as in a family's pet bedding, where the litter or bedding comes into contact with the animal secretions and excretions, an odor problem exists. Most solutions to this problem are currently remedied by the "masking" technique with inadequate results. The same problems as discussed previously are present with products used to mask odors of this nature.

Another area where the problem of odors is apparent is in bathrooms. The problem exists in both public and private (or home) bathrooms. Associated with this is the odor problem that exists in lockerrooms. On a larger, industrial scale serious odor problems exist in waste water or sewage treatment plants and in and around chemical or processing factories. The masking technique or remedy has been, and is currently, being used in these situations with the associated problems as previously discussed.

A further odor problem that is universally considered offensive is that associated with urinary or fecal matter of humans or animals. When not associated with litter or bedding the odor is extraordinarily

2

pervasive and pungent. Masking is the most commonly used method to control such odor and has not been effective.

A product that would eliminate these types of odors would have immediate applicability in the following, but not limiting, areas: floors, floor coverings and substrates of barns, chicken houses, chicken coops, pig sties, farrowing houses, milking facilities, holding pens, cages, terrariums, aviaries, poultry houses, zoo cages and exhibits, reptile and amphibian cages and herpeterriums, transportation trailers and trucks, corrals, kennels, catteries, rodent cages, rodeo chutes, horse arenas, homes, offices, diapers, diaper pails, urinals, toilets, bathroom floors, animal bedding, animal litter wastes, human and animal urine, human and animal feces, industrial ammonia products and facilities associated therewith, lockerrooms, farms, waste water and sewage facilities, and incorporation into human deodorants.

Accordingly, a safe and effective product that eliminates odors caused by fecal, urinary or glandular matter; chemical process by-products or waste products; and human body odor caused by bacterial action on glandular secretions is needed to solve the problem associated with such odors and to overcome the drawbacks associated with the currently prevailing method of masking the odors. A goal of this invention is to fulfill this need in the odor control industry.

More specifically, an object of the invention is to provide a product and method that eliminates odors by directly acting with the chemical compounds that cause the odor.

Another object of the invention is to provide a product that eliminates the odor in an efficient manner so that continual reapplication to control the same odor is not necessary.

Another object of this invention is to provide a product and method of odor removal that is safe to humans and animals.

Yet another object of this invention is to provide a product and method of odor removal that is inert and non-toxic to the products that make up animal bedding and litter.

An additional object of this invention is to provide a product and method of odor removal that is adaptable to large scale, industrial use or for individual, home use.

Another object of the invention is to provide a product and method of odor removal that can be utilized in either a liquid or solid form.

A further object of this invention is to provide a product and method of odor removal that does not react with typical quaternary ammonium compound disinfectants or solid diluents such as sodium bicarbonate, sodium chloride and sodium sulfate.

Another object of this invention is to provide a product and method of odor removal that can be combined with non-aqueous liquid dispersants without reaction or diminished usefulness.

An additional object of this invention is to provide a product and method of odor removal that eliminates the odors instantaneously and permanently.

A further object of the invention is to provide a product and method of odor removal that is not affected by various or changing environmental conditions.

Yet a further object of this invention is to provide a product and method of spill control (including containment and subsequent clean-up) for chemicals, such as ammonia and amines and compounds and mixtures thereof, that will control the, often highly toxic, odors.

Other and further objects of the invention, together with the features of novelty appurtenant thereto, will appear in the course of the following description of the invention.

I have discovered that an alkali metal formaldehydebisulfite effectively eliminates odors caused by matter of fecal, urinary, and glandular origin such matter normally consisting of ammoniacal substances (consisting of ammonia, amines, amides and other nitrogenous substances), sulfur-containing substances (such as mercaptans and thiols), and various musks. A pure alkali metal formaldehydebisulfite, a mixture of alkali metal formaldehydebisulfites, a mixture of one or more alkali metal formaldehydebisulfites with various diluents, carriers or other ingredients, or a mixture of those chemical substances (i.e. alkali metal sulfites, bisulfites, metabisulfites, or thiosulfates with formaldehyde or paraformaldehyde) in pure or mixed states, with or without diluents, carriers or other ingredients can be utilized directly on the odor causing substance or carrier substance to eliminate by chemical reaction any associated odor. The odor eliminating product can be manufactured either in a dry form (i.e., powder, granule, flake, tablet, cake, pellet, bolus, capsule, briquette) with or without additives, or in a water solution with or without other dissolved or suspended substances.

In summary, the invention relates to a process for eliminating odors caused by substances containing ammonia, amines, amides and other nitrogenous compounds, mercaptans, thiols and other sulfur-containing substances, or various musks. The alkali metal formaldehydebisulfite is selected from the group consisting of sodium formaldehydebisulfite and potassium formaldehydebisulfite. In a preferred embodiment, the alkali metal formaldehydebisulfite is sodium formaldehydebisulfite added, unadulterated as a powder, to fresh litter in the proportion of 1 gram of sodium formaldehydebisulfite per 0.5 kilograms of litter.

Unless otherwise stated herein, indication of parts or percentages are given on a weight basis.

I have discovered that an alkali metal formaldehydebisulfite is unexpectedly effective and safe to eliminate odors caused by substances containing ammonia, amines, amides and other nitrogenous substances, mercaptans, thiols and other sulfur-containing substances, and various musks. A pure alkali metal formaldehydebisulfite, a mixture of alkali metal formaldehydebisulfites, a mixture of one or more alkali metal formaldehydebisulfites with various diluents, carriers or other inert ingredients, or a mixture of substances which, upon chemical reaction, produce an alkali metal formaldehydebisulfite can be utilized directly in dry or solution form on the odor causing substance, or the odor carrying substance, to eliminate by chemical reaction the odors associated therewith.

Neither the reaction mechanism, nor the reaction products, by which the phenomena of eliminating odors from substances of the type described above with an alkali metal formaldehydebisulfite is understood. Experimental research, however, shows that odors are controlled by application of sodium formaldehydebisulfite to odor causing substances. The results show that in field tests when sodium formaldehydebisulfite in any of its various forms is applied to any of the odor causing substances discussed previously to eliminate the odor emanating therefrom, the odor is eliminated, neutralized or controlled as determined by both subjective odor tests and direct measurement of concentrations the odorous substances in the air.

As used herein, the terms remove, eliminate, neutralize or control are used interchangeably to refer to the discovered ability of alkali metal formaldehydebisulfites to render odorless animal or human substances of fecal, urinary and glandular origin or other substances containing ammonia, amines, amides and other nitrogenous substances, mercaptans, thiols and other sulfur-containing substances, or various musks.

The alkali metal formaldehydebisulfites useful in this invention include sodium formaldehydebisulfite (hereinafter referred to as "SFB" and potassium formaldehydebisulfite (hereinafter referred to as "KFB"). The compound SFB has the chemical formula $HOCH_2SO_3Na$, and is also known as formaldehyde sodium bisulfite and sodium hydroxymethane sulfonate. The compound KFB has the chemical formula $HOCH_2SO_3K$, and is also known as formaldehyde potassium bisulfite and potassium hydroxymethane sulfonate.

The alkali metal formaldehydebisulfite may be utilized in the dry form with a variety of inert materials such as diluents, carriers, excipients, lubricants, disintergrants, colorants, disinfectants and mixtures thereof. A diluent (i.e., tricalcium phosphate) is an inert material to achieve a desirable and beneficial effect. A carrier (i.e., salt) is an inert material used to deliver or disperse an active material. Suitable diluents and carriers for use with alkali metal formaldehydebisulfites include salt and other similar, nonreactive, pH-neutral electrolytes and insoluble salts such as starch, sugars, clays, and calcium sulfate. An excipient (i.e., starch) is an inert material used as a binder in tablets. Suitable excipients for use with alkali metal formaldehydebisulfites include polymers and gums such as cellulose gum and povidone, and starches. A lubricant (i.e., magnesium stearate) is an inert material used to reduce friction during filling or tableting processes. Suitable lubricants for use with alkali metal formaldehydebisulfites include fatty acid salts such as calcium stearate or magnesium stearate, and paraffinic compounds and fatty acids such as paraffin wax and stearic acid. A disintergrant is an inert material that causes tablets and boluses to burst upon exposure to appropriate conditions. Suitable disintergrants for use with alkali metal formaldehydebisulfites include polymers such as cross-linked povidone, and effervescent mixtures such as sodium bicarbonate/citric acid. A colorant is an inert material that imparts color to another material or mixture. Suitable colorants for use with alkali metal formaldehydebisulfites include lakes (i.e., organic pigments on an adsorptive inorganic substrate) such as rose madder, and non-oxidizing dyes such as acriflavine. A disinfectant is typically a quaternary ammonium compound used to disinfect and kill bacteria. Suitable disinfectants for use with alkali metal formaldehydebisulfites include benzalkonium chloride, methylbenzethonium chloride, benzethonium chloride, and dimethyl-dibenzyl-ammonium chloride.

The alkali metal formaldehydebisulfites are also inert with respect to most animal litter substrates. Typical animal litter substrates include typical clay, clay-like, and natural zeolite materials (i.e., montmorillenite and clinoptilolite) and cellulose-based litters (i.e., alfalfa pellets, wood chips and sawdust, and paper.

The pure dry active alkali metal formaldehydebisulfite may be packaged in containers such as bottles, boxes, non-porous bags, and drums that serve to protect the integrity of the product and to allow for appropriate dispensing. Other dry forms of the product for use in this invention include unit dose tablets, capsules, boluses or packets. Individual dosage units may be weighed or measured by bulk volume if supplied in the form of powders, granules, pellets, or flakes. Larger unit dose requirements, such as for barns, chemical factories or sewage or waste-water facilities, may be applied as a dry manufactured cake.

The alkali metal formaldehydebisulfite may be used directly for odor control. No attempt has been made to relate the quantity of SFB required to control or neutralize the offensive odor. As with many process chemicals, however, proper performance of a consumer formulation is often best achieved by providing a dosage form that is easily applied and only requires commonly available volumetric measuring devices such as teaspoons (1/6 fl. oz., 4.93 ml), tablespoons (1/3 fl. oz., 9.86 ml) or cups (8 fl. oz., 236.64 ml). Although weight measurement of solids is routinely very accurate, it is quite uncommon for the consumer to employ such measurements especially as to a totally new concept of eliminating odors when the proper amount to use is not commonly known to the consuming public. The use of pre-weighed unit dosages of a pure substance or its mixtures in the form of tablets, boluses, capsules or packets is the typical and preferred method to deliver dry forms for applications of this invention such as ammonia by-products of chemical factories and waste water or sewage treatment plants. Since this invention has such a variety of uses, the many varieties of dosage forms is extremely important and ultimately helpful to the consumer. Many of the applications of this invention will require use of the SFB in a powder form and application will be directly to the odor causing substance by means of a shake bottle. Equally satisfactory, however , is a formulation as a dry powder or granular mixture of a consistency that readily lends itself to accurate and repeatable volumetric dosage measurements.

To illustrate the foregoing principles with respect to suitable formulations for application to animal litter boxes, the following dry forms of product represent convenient formulations adapted for easy use by the lay consumer:

1. 1 gram of unadulterated SFB powder mixed with 0.5 kg of typical clay litter.

2. A mixture of SFB and sodium bicarbonate (1:9 by weight) where 10 grams of the mixture is mixed with 0.5 kg of litter.

3. An aqueous solution of SFB (21% by weight).

4. An aqueous solution of SFB (24% weight/volume).

5. An equimolar mixture of sodium sulfite and paraformaldehyde designed to be dissolved in water to produce a solution of SFB.

In the various product formulations of this invention, the usual practices of cleanliness and sanitation are to be followed as these relate to the conditions of mixing, packaging and storage. The purity and grades of the various ingredients including formaldehyde gas, formaldehyde solution, sodium bisulfite, sodium formaldehydebisulfite, potassium formaldehydebisulfite, methanol, salt starch, tableting excipients, tableting lubricants, cross-povidone, buffers and their components, colorants and dyes, diluents and carriers, and water may vary from standard commercial or technical grades to the highly purified, reagent or pharmaceutical grades. The physical forms of the various solid components may vary from ultra-fine powders to granular and flake forms. Liquid components should be free from suspended or precipitated material, but should such material be present it should be removed by suitable settling and decantation or filtering. Any water should be similarly free of suspended or precipitated material as well as free from free or combined chlorine, including chloramines, and free or ionized ammonia. In addition, the water must be free of other free halogens such as iodine (I) and bromine (Br) and their combined forms which may reduce the final required concentration of the SFB. Neutralization or removal of potentially interfering substances would be permissible prior to the addition or formation of the SFB. With observance of the foregoing, any potable water source may be used in the manufacturing process.

All components used in the production of these odor controlling products must be free from, or rendered free from, substances that may be toxic or otherwise detrimental to humans, animals and also inert with respect to natural and synthetic fabrics, porcelain, aluminum and other metals, tile, clay, sawdust, paper, etc.

Both acids and alkalies hasten the decomposition of the SFB. Therefore, solutions of the SFB must be manufactured so that the final pH lies between 6.0 and 8.0. Dry formulations must be manufactured so that if the resultant mixture is hygroscopic, then decomposition of the SFB will not result due to an acidic or alkaline environment being created within the mixture.

In typical production of a liquid form of this invention, the following parameters should be considered preferable but not essential. If dry SFB is to be used it should be of photographic grade that is typically less than 0.1% free formaldehyde and less than 0.1% uncombined sodium bisulfite and of powder form. If the SFB is to be formed by reaction, then 35-38% formaldehyde solution with no more than 15% methanol can be reacted with photographic grade, powdered or granular sodium bisulfite. Water should be completely deionized and have a pH of 6.5 to 7.5. The final solution is to be clear, colorless and free from suspended or precipitated matter. Mixing and filling equipment may desirably be fabricated from stainless steel or polyvinyl chloride. The liquid product may be packaged and stored in polyethylene containers with polyethylene or polypropylene closures.

Due to the formation hydroxide ions ($OH^-$), the in situ reaction of formaldehyde solution or paraformaldehyde solution with alkali sulfites or thiosulfates to form formaldehydebisulfites would not usually be recommended. However, in instances where the formaldehydebisulfites thus formed would be reacted with ammonia, amines, amides and other nitrogenous substances in such a manner that the alkaline decomposition of the formaldehydebisulfites would not occur, or would not occur to an extent great enough to mitigate the desired results, such in situ formation of formaldehydebisulfites could be used to advantage.

As previously indicated, the reaction mechanism and reaction products are not understood in the reaction of alkali metal formaldehydebisulfite with odor causing substances such as urinary, fecal, or glandular matter. What is known is that these types of matter partially consist of ammoniacal substances, sulfur-containing substances and musks and that these substances are, either directly, or upon subsequent chemical change, capable of producing ammonia, amines, other ammoniacal or ammonia-or amine-like odors, thiols, mercaptans, other sulfurous odors, musk or musk-odors. Research relating to the control of these odors has been both subjective and objective (quantitative) in nature. No attempt has been made to relate the odor emanating from these types of products with the quantity of urinary, fecal or glandular matter deposited. Nor has any attempt been made to evaluate the exact chemical nature of the odors emanating from the test subject products.

The invention is further exemplified with reference to the following research examples investigating the effect of SFB for use in animals' litter to control the odor associated therewith.

EXAMPLE 1

Field experiments were conducted on homes and pet shops to evaluate the odor controlling capabilities of SFB when applied to animals' litter. The following animals' litter have been tested: adult cats, kittens, Guinea pigs, and rabbits. The animal's weights have ranged from 2.7 to 4.5 kg for adult cats, 0.5 to 1.0 kg for kittens, 0.5 to 1.0 for Guinea pigs, and 1.8 to 5.4 kg for rabbits. The animals were fed their normal diet and all conditions were those normally associated with a home pet for those studies done at home and those normal for a pet shop for those studies done at the pet shop.

The purpose of this test was to control odor resulting from fresh, aged, or decomposing matter of fecal, urinary and glandular origin in litter and enclosures used for the keeping, breeding, or transportation of animals.

The litter in use in these experiments were the typical clay, clay-like, and natural zeolite materials (i.e., montmorillenite and clinoptilolite) and cellulose-based litters (i.e., alfalfa pellets, wood chips and sawdust, and paper).

Odor control was achieved in home field tests of cat litter boxes containing typical clay litter under the following conditions:

1. Mixing 1 gm of unadulterated SFB powder per 0.5 kg litter;

2. Mixing 10 gm of a SFB and sodium bicarbonate mixture (1:9 by weight) per 0.5 kg of fresh or used, and cleaned to remove feces, litter;

3. Spraying the surface of fresh or cleaned litter in litter boxes with an aqueous solution of SFB (21% by weight) at a rate ranging from 0.729 ml to 0.833 ml per application (0.176 to 0.201 gm SFB) to litter in litter containers with surface areas ranging from 972 to 2444 $cm^2$ (140 to 352 $in^2$); averaging 0.000207 g SFB/ $cm^2$ (0.00144 g SFB/ $in^2$).

Odor control was achieved in pet shop field tests of kitten, rabbit, and Guinea pigs litter using typical litter of the type described in litter containers of 777 $cm^2$ surface area by spraying fresh litter every 24 hours with 1.042 ml of SFB (24 % weight/volume). The litter was replaced every 24 hours with fresh litter and the fresh litter sprayed with the SFB solution and the top wetted layer was mixed throughout the remaining litter in the container. Odor was controlled for the entire 24 hour period. The application rate of SFB was equivalent to 0.00147 $g/cm^2$ (0.00102 $g/in^2$).

Odor control was further achieved in pet shop field tests of standing urine puddles in kitten, rabbit, and Guinea pig cages when an aqueous solution of SFB (24% weight:volume) was sprayed ad libitum directly onto the urine puddles. The urine puddles were not in contact with the treated litter. This ad libitum spraying method onto fresh unabsorbed urine resulted in complete control of odors that would have ordinarily emanated from such urine.

EXAMPLE 2

An experiment was conducted in a working swine nursery. The nursery housed approximately one hundred piglets of similar age (approximately three months). The structure of the nursery was situated over a collection pit which was covered over by "T-bar" flooring upon which the piglets ate, slept, defecated and urinated. The flooring and pit construction was typical of these types of structures, and the wastes dropped directly into the pit through slots in the flooring. It was estimated that the pit had a capacity of approximately 39,000 gallons and was approximately 75% filled with liquid. The odor within the nursery was typical of this type operation and a major component was obviously ammonia and/or amines.For this test a commercially available dosimeter tube designed for the detection of ammonia and amines in air was opened and positioned in the central part of the nursery. The dosimeter tube consists of glass tube with external, linear measurements (0 to 60 mm) printed on it and a strip of indicating paper impregnated with bromophenol blue. The dosimeter tube provides a quantitative method for estimating the concentration of ammonia and amines in the surrounding air. The measurement being indicated by th  :vance of the blue color along the length of the paper strip in the tube as compared to the printed measu.ements on the tube. The major reason for using the dosimeter was that the odor was so strong that a meaningful quantification of its reduction would be difficult without performing a series of tests to determine the nature and concentrations of each component of the gasses escaping from the pit. No attempt was made to completely eliminate the nursery odor, instead it was a test to determine whether or not any measurable reduction in the ambient level of one component of the odoriferous substances could be achieved and measured by some method other than by subjective methods.

This test was conducted in February of 1987 during a time of the winter in which the nursery was subject to the least amount of ventilation. While it is recognized that the ammonia in the nursery air is quite detrimental to the general health of the piglets (causing a condition similar to pneumonia such as the loss of beneficial cilliary activity in the bronchial apparatus), it is often considered less harmful than subjecting the juvenile swine to temperature variations and wind drafts that would accompany unheated ventilation heavy enough to refresh the air in the nursery and remove the odor.

In this test twenty-five pounds (25 lb.) of dry, powder SFB were added directly to the pit, through the "T-bar" flooring, in the north-west corner of the nursery, and the residual powder was rinsed into the pit with five gallons of tap water from a bucket. Prior to the addition of the SFB to the pit, a dosimeter tube was opened and placed in a preselected position about 5 feet above the floor and in the center of the room and monitored for forty-eight hours. The 24-hour reading was 43 mm, the 48-hour reading was 60 mm (60 mm representing 100% color change of the dosimeter color strip). According to the literature accompanying the dosimeter tube these readings are equivalent to ammonia concentrations of 13.85 ppm, and 12.5 ppm for an average of 13.18 ppm.

After addition of the SFB to the pit a new dosimeter tube was placed in the same location as the first one and the following readings were obtained: 24-hour reading = 19 mm and 48-hour reading = 33 mm. These readings are equivalent to 3.589 ppm and 4.400 ppm respectively, with an average of 3.994 ppm.

The calculation of the preceding concentrations was done with the following equation:

$$C = 15S + \frac{S^2}{kt}$$

where: C = concentration in ppm

S = stain length (in the dosimeter tube) in mm

k = value taken from dosimeter literature

t = sampling time in hours

The results of this test indicate that removal of ammonia and/or amines from swine nursery pits is not only practical but if one assumes an arbitrary cost of three-dollars per pound for SFB it is also economically feasible. At a cost of $3.00/lb the cost of reducing the ambient ammonia-equivalent concentration was $75.00 or approximately $8.82/ppm (of reduction). With the use of dosimeter tubes a farmer could easily monitor the nursery air for and indication of when to add the SFB to the pit to control the build-up of ammonia and/or amines. Furthermore, with a minimum of record keeping, a farmer could determine the exact quantity of SFB needed to be metered, on a continuous basis, into a given waste pit for a given population and age of piglets being housed. The cost of the SFB would be partially or completely offset by the saving in healthier livestock and lower utilities' cost (heating and ventilation).

## EXAMPLE 3

A small-scale chemical spill was simulated in an experiment designed to test not only odor control but also containment and subsequent clean-up and removal. In this test two liters of stronger ammonia water (26° Be, 28% $NH_3$ in water) were poured onto the floor of a laboratory with restricted ventilation. Five minutes later enough dry powder SFB was applied to the "spill" in a manner which prevented the spread of the liquid, then enough SFB was applied directly to the "spill" area so that the liquid was completely absorbed by the powder and so that the powder in the center of the "spill" area was dry for a minimum of five minutes.

The application described above was achieved by spreading the SFB powder with a plastic scoop around the edges of the "spill" at a width of approximately two-inches (2 in). Then the "spill" itself was covered over with SFB from the scoop to an average, estimated depth of one-quarter of an inch (0.25 in), followed by the application of additional SFB powder from the scoop as needed to achieve a dry (by visual inspection) covering to the entire "spill" area. The complete "spill" containment was achieved in approximately 2 minutes.

After allowing the contained/absorbed "spill" mass to remain on the floor for three hours subsequent clean-up and removal was easily achieved by sweeping the resultant mass into a hand-held dust pan with a whiskbroom. The residue left adhering to the floor's surface was easily removed with a moistened paper towel. The sweepings and toweling were placed into a standard plastic trash bag for disposal in a waste receptacle.

Following the initial release of gaseous ammonia from the fresh "spill" into the atmosphere of the laboratory the odor quickly dissipated and was completely discharged within thirty minutes of the completion of the spill containment and absorption. The quantity of SFB required for the "spill" containment and absorption was approximately two kilograms, or one kilogram per liter of "spill". This value, of course, will vary from spill to spill and will be dependent no only upon the volume of the spilled liquid but also the surface area covered by the spill. In spill containment and control, however, the relative cost of the counteractive agents are of minor concern, especially where human health and safety are of immediate concern.

In a comparison test identical quantities of stronger ammonia water were again "spilled" and its containment and absorption were attempted with a fine granular ammonia-absorbing, natural zeolite (clinoptilolite) in a manner identical to that used in the test with SFB. In this test containment was easily accomplished as was subsequent absorption of the liquid. However, after three hours the perceived ammonia odor was little changed in the laboratory.

In both tests the observer left the laboratory following the "spill" containment/absorption, and returned at intervals of fifteen minutes, one-half hour, two hours and three hours.

## EXAMPLE 4

A series of experiments were conducted at the Laboratory Animal Center of the University of Missouri, Kansas City. These experiments were conducted in an isolation room housing adult white rabbits in stainless steel cages. Each cage was equipped with a bottom tray containing wood shavings litter. The litter tray was situated below a wire floor in the cage so that the rabbits did not come into contact with the litter, and so that both urine and feces dropped through the cage floor onto the litter below.

Four cages, randomly selected, were cleaned and the litter trays were emptied, cleaned and replaced with fresh, dry litter. In this test, which was designated the "control test", the same commercially available dosimeter tubes, as described in Example 2, were used to determine a "base level" of air borne ammonia and amines emanating from the litter over a seven-day period. The four selected cages represented only twenty-five percent (25%) of the total number of cages in the room. A fifth dosimeter tube was attached to the wall of the room in which the cages were housed.

At the end of the control test, the cages were again cleaned, and the litter was replaced as before. In addition new dosimeter tubes were put in place in the identical locations used in the control test. This next test series was designated the "spray trial test". The spray trial test was conducted by spraying only those areas on the litter where the rabbits had urinated. The spraying was done at the end of each twenty-four hour period (except for the last period). The spray was a 21% (w/v) solution of SFB in water. Rabbits have a well-known habit of urinating in only one or two places where housed in a cage; thus the significance of spraying only the areas where the rabbits had urinated, and not the entire litter surface area as in Example 1. In both tests the dosimeter readings were recorded at the end of each twenty-four hour period. The readings are tabulated below (Tables 1 and 2).

### Table 1.  Control Test Dosimeter Stain Lengths

| Dosimeter Placement: | 24 Hrs. | 48 Hrs. | 72 Hrs. | 96 Hrs. | 120 Hrs. | 144 Hrs. | 168 Hrs. |
|---|---|---|---|---|---|---|---|
| Cage A | 0 mm | 0 mm | 0 mm | 0 mm | 23 mm | 24 mm | 32 mm |
| Cage B | 0 mm | 0 mm | 0 mm | 0 mm | 20 mm | 20 mm | 31 mm |
| Cage C | 0 mm | 0 mm | 0 mm | 0 mm | 22 mm | 23 mm | 30 mm |
| Cage D | 0 mm | 0 mm | 0 mm | 0 mm | 26 mm | 28 mm | 37 mm |
| Room E | 0 mm | 0 mm | 0 mm | 0 mm | 17 mm | 17 mm | 24 mm |

### Table 2.  Spray Trial Test Dosimeter Stain Lengths

| Dosimeter Placement: | 24 Hrs. | 48 Hrs. | 72 Hrs. | 96 Hrs. | 120 Hrs. | 144 Hrs. | 168 Hrs. |
|---|---|---|---|---|---|---|---|
| Cage A | 0 mm | 0 mm | 0 mm | 0 mm | 11 mm | 17 mm | 21 mm |
| Cage B | 0 mm | 0 mm | 0 mm | 0 mm | 15 mm | 20 mm | 33 mm |
| Cage C | 0 mm | 0 mm | 0 mm | 0 mm | 15 mm | 20 mm | 26 mm |
| Cage D | 0 mm | 0 mm | 0 mm | 0 mm | 17 mm | 19 mm | 27 mm |
| Room E | 0 mm | 0 mm | 0 mm | 0 mm | 9 mm | 16 mm | 21 mm |

In both tests the dosimeter tubes failed to record any changes in ammonia and amines until the one-hundred and twenty hours point. This was probably due to an "incubation period" during which mineralizing microorganisms (i.e. bacteria and fungi) had not yet reached a population level significant enough to liberate measurable ammonia or amines from the nitrogenous components of the rabbits' urine (i.e. urea, uric acid and proteins).

Applying the same calculation as used in Example 2 the dosimeter readings were converted into parts per million of ammonia (ppm $NH_3$). These calculations are tabulated below (Tables 3 and 4):

Table 3: Control Test Ammonia (Equivalent) Concentrations

| Dosimeter Placement: | 120 Hours | 144 Hours | 168 Hours |
|---|---|---|---|
| Cage A | 0.971 ppm | 0.867 ppm | 1.190 ppm |
| Cage B | 0.778 ppm | 0.648 ppm | 1.130 ppm |
| Cage C | 0.904 ppm | 0.809 ppm | 1.070 ppm |
| Cage D | 1.180 ppm | 1.110 ppm | 1.530 ppm |
| Room E | 0.604 ppm | 0.504 ppm | 0.743 ppm |

Table 4: Spray Trial Test Ammonia (Equivalent)
Concentrations

| Dosimeter Placement: | 120 Hours | 144 Hours | 168 Hours |
|---|---|---|---|
| Cage A | 0.318 ppm | 0.504 ppm | 0.600 ppm |
| Cage B | 0.500 ppm | 0.648 ppm | 1.260 ppm |
| Cage C | 0.500 ppm | 0.648 ppm | 0.846 ppm |
| Cage D | 0.604 ppm | 0.598 ppm | 0.900 ppm |
| Room E | 0.240 ppm | 0.459 ppm | 0.600 ppm |

Table 5: Control Test Ammonia (Equivalent) Concentration
Averages and Means

| Dosimeter Placement: | Average | Mean |
|---|---|---|
| Cage A | 1.010 ppm | 1.030 ppm |
| Cage B | 0.853 ppm | 0.890 ppm |
| Cage C | 0.928 ppm | 0.940 ppm |
| Cage D | 1.270 ppm | 1.320 ppm |
| Room E | 0.617 ppm | 0.623 ppm |

Table 6: Spray Trial Test Ammonia (Equivalent)
Concentration Averages and Means

| Dosimeter Placement: | Average | Mean |
|---|---|---|
| Cage A | 0.474 ppm | 0.459 ppm |
| Cage B | 0.802 ppm | 0.879 ppm |
| Cage C | 0.665 ppm | 0.673 ppm |
| Cage D | 0.701 ppm | 0.749 ppm |
| Room E | 0.433 ppm | 0.420 ppm |

Table 7: Control Test Time Period Ammonia (Equivalent)
Concentration Averages and Means (Cages Only)

| Time Period: | Average | Mean |
|---|---|---|
| 120 hours | 0.959 ppm | 0.981 ppm |
| 144 hours | 0.860 ppm | 0.881 ppm |
| 168 hours | 1.230 ppm | 1.300 ppm |

Table 8: Spray Trial Test Time Period Ammonia (Equivalent)
Concentration Averages and Means (Cages Only)

| Time Period: | Average | Mean |
|---|---|---|
| 120 hours | 0.481 ppm | 0.461 ppm |
| 144 hours | 0.599 ppm | 0.576 ppm |
| 168 hours | 0.901 ppm | 0.929 ppm |

In the following Table 9 there is shown the percent of reduction of the ammonia equivalent concentrations as calculated from the concentration averages for each of the cages and the room in accordance with the formula:

$R = 100(Ac - As/Ac)$

where: $R$ = Percent Reduction
$Ac$ = Average Concentration (in ppm) of Control Test
$As$ = Average Concentration (in ppm) of Spray Trial Test

Table 9: Percentage of Ammonia (Equivalent)
Concentration Reductions

| Dosimeter Placement: | Reduction: |
|---|---|
| Cage A: | 53.1% |
| Cage B: | 55.1% |
| Cage C: | 28.4% |
| Cage D: | 45.0% |
| Room E: | 29.8% |

In the following Table 10 there is shown the reduction percentages for the cages for each of the time periods; calculated as follows:

12

Rt = 100(Act - Ast)/Act
where: Rt = Percent Reduction
 Act = Average Concentration (in ppm) of Control Test Time Periods
 Ast = Average Concentration (in ppm) of Spray Trial Test Time Periods

## Table 10: Percentage of Ammonia (Equivalent) Concentration Reductions for each Time Period (cages only)

| Time Period: | Reduction: |
|---|---|
| 120 hours | 49.9% |
| 144 hours | 30.3% |
| 168 hours | 26.8% |

These tests were significant from the standpoint that the absolute odor of emanating from the rabbit cages is routinely quite low, and the center's staff reported that ammoniacal odors are not perceived as being a problem. The dosimeters, however, did indicate the presence of measurable levels of ammonia and/or amines.

EXAMPLE 5

In this trial a prepackaged, compressed paper and wood chip litter pad (which is designed to be shredded by small caged rodents like hamster and gerbils) was treated, respectively, with a solution of SFB. The purpose of this trial was to determine whether or not the structural integrity of the litter would be maintained under conditions of treatment and subsequent drying.

The litter pad, which weighed 14.12 grams, was saturated with a solution of SFB in water (21% w/v) by soaking the pad in an excess volume of the solution. After saturating the pad was reweighed and was demonstrated to have absorbed slightly more than its own weight of the solution; the saturated pad weighed 28.69 grams.

Next the pad was dried at 105° C for 12 hours. The resulting pad was not dissimilar in appearance to its original, untreated state. There was no odor to the pad, and no discoloration had occurred. In addition there was no evidence that the pad had been treated at all.

Prior testing designed to determine the toxicity of ingested SFB to rats, and eye and skin irritation to rabbits suggests that its use in bedding and litter will be safe. The oral toxicity of a 21% (w/w) solution to rats, for instance, has been demonstrated to be greater than 5,000 milligrams per kilogram of (rat) body weight. Therefore treated litter and bedding that is shredded by, or carried in, the mouths of the animals would not be directly or indirectly injurious to the animals.

As indicated, this invention provides a quick, complete method to control odors emanating from odor-causing substances, containing ammonia, amines, amides and other nitrogenous substances, mercaptans, thiols and other sulfur-containing substances, and various musks. The invention eliminates odors completely when applied as either a dry form or an aqueous solution. The invention is non-toxic to humans and animals. It controls odor when applied directly to the odor-causing substance or when mixed with the odor carrying substance, such as litter. It can easily be combined with known disinfectants to provide odor control and disinfection and sanitization.

From the foregoing it will be seen that this invention is one well adapted to attain all the ends and objects hereinabove set forth, together with the other advantages that are obvious and that are inherent to the invention.

It will be understood that certain features and subcombinations are of utility and may be employed without reference to other features and subcombinations. This is contemplated by and is within the scope of the claims.

Since many possible embodiments may be made of the invention without departing from the scope thereof, it is understood that all matter herein set forth or shown in the research examples is to be interpreted as illustrative and not in a limiting sense.

## Claims

1. A process for controlling an odour of a nitrogenous or sulfur-containing compound, a musk and/or an odour of fecal, urinary or glandular origin, which comprises applying to the odour, its source or the area of its source a bisulfite selected from sodium formaldehydebisulfite and potassium formaldehydebisulfite, or material which reacts to form the bisulfite in situ.

2. A process according to claim 1, wherein sodium formaldehydebisulfite is applied.

3. A process according to claim 1, wherein a formaldehyde and water solution, and a sodium bisulfite and water solution (which together produce sodium formaldehydebisulfite) are applied.

4. A process according to claim 3, wherein the formaldehyde and water solution comprises at least 9% by weight formaldehyde.

5. A process according to claim 3 or claim 4, wherein the sodium bisulfite and water solution comprises at least 33% by weight sodium bisulfite.

6. A process according to any of claims 3 to 5, wherein the bisulfite moiety is derived from an alkali metal sulfite, metabisulfite or thiosulfate.

7. A process according to any preceding claim, wherein the bisulfite or reactive material is applied directly to the odour-carrying or odour-causing substance.

8. A process according to claim 7, wherein the odour-carrying substance is animal litter (e.g. clay, natural zeolite or cellulose-based litter), animal bedding or an animal cage or container.

9. A process according to any preceding claim, wherein the bisulfite is applied in combination with inert material selected from diluents, carriers, excipients, lubricants, disintegrants, colorants and disinfectants.

10. A process according to claim 9, wherein any diluents, carriers or excipients are selected from salt, sodium sulfate, potassium chloride, starch, sugars, clays, calcium sulfate, tricalcium phosphate, calcium silicate, fumed silica, algin, alginates, natural gums, cellulose gum and povidone, wherein any lubricants are selected from calcium stearate, magnesium stearate, aluminium stearate, zinc stearate, paraffin wax and stearic acid, wherein any disintegrants are selected from cross-linked povidone, sodium bicarbonate/citric acid and modified starches, and wherein any disinfectants are selected from benzalkonium chloride, methylbenzethonium chloride, benzethonium chloride and dimethyldibenzylammonium chloride.